# FASCICULE DE BREVET EUROPEEN

(11) **EP 1 496 884 B1**
(45) Date de publication et mention de la délivrance du brevet: **14.06.2006**
(21) Numéro de dépôt: 03745819.7
(22) Date de dépôt: 04.04.2003
(51) Int. Cl.: A61K 31/196, A61K 31/616, A61P 7/02

(54) **ASSOCIATION D'UN ANTITHROMBOTIQUE ET D'ASPIRIN ET SON UTILISATION POUR LE TRAITEMENT DES MALADIES THROMBOTIQUES ATHEROSCLEREUSES**
VERBINDUNG EINES ANTITHROMBOTISCHEN MITTELS MIT ASPIRIN UND DESSEN VERWENDUNG ZUR BEHANDLUNG VON ATHEROTHROMBOTISCHEN ERKRANKUNGEN
ASSOCIATION OF AN ANTITHROMBOTIC AGENT WITH ASPIRIN AND USE THEREOF FOR TREATING ATHEROTHROMBOTIC DISEASES

(30) Priorité: 05.04.2002 FR 0204222
(43) Date de publication de la demande: 19.01.2005
(73) Titulaire: Les Laboratoires Servier, 92415 Courbevoie Cedex (FR)
(72) Inventeur: VERBEUREN, Tony, F-78540 Vernouillet (FR); LAVIELLE, Gilbert, F-78170 La Celle Saint Cloud (FR); CIMETIERE, Bernard, F-75020 Paris (FR); VALLEZ, Marie-Odile, 93100 Montreuil (FR)
(86) Numéro de dépôt international: PCT/FR2003/001054
(87) Numéro de publication internationale: WO 2003/084525

(56) Documents cités:
- EP-A- 0 648 741
- CIMETIÈRE B, DUBUFFET T, LANDRAS C, DESCOMBES J-J, SIMONET S, VERBEUREN TJ, LAVIELLE G: "New tetrahydronaphthalene derivatives as combined thromboxane receptor antagonists and thromboxane synthase inhibitors" BIOORGANIC & MEDICINAL CHEMISTRY LETTERS, vol. 8, 1998, pages 1381-1386, XP002223657
- MUTSCHLER E, GEISSLINGER G, KROEMER HK, SCHÄFER-KORTING M: "Mutschler Arzneimittelwirkungen - Lehrbuch der Pharmakologie und Toxikologie, 8. Auflage" 2001 , WISSENSCHAFTLICHE VERLAGSGESELLSCHAFT MBH , STUTTGART XP002223660 page 231, alinéa 1
- CIMETIERE BERNARD ET AL: "Synthesis and biological evaluation of new tetrahydronaphthalene derivatives as thromboxane receptor antagonists." BIOORGANIC & MEDICINAL CHEMISTRY LETTERS, vol. 8, no. 11, 2 juin 1998 (1998-06-02), pages 1375-1380, XP002223659 ISSN: 0960-894X
- SIMONET S, DESCOMBES J-J, VALLEZ M-O, DUBUFFET T, LAVIELLE G, VERBEUREN TJ: "S 18886, A New Thromboxane (TP)-Receptor Antagonist is the Active Isomer of S 18204 in All Species, Except in the Guinea-Pig" ADVANCES IN EXPERIMENTAL MEDICINE AND BIOLOGY, vol. 433, 1997, pages 173-176, XP008010971 US
- CAYATTE ANTONIO J ET AL: "The thromboxane receptor antagonist S18886 but not aspirin inhibits atherogenesis in apo E-deficient mice: Evidence that eicosanoids other than thromboxane contribute to atherosclerosis." ARTERIOSCLEROSIS THROMBOSIS AND VASCULAR BIOLOGY, vol. 20, no. 7, juillet 2000 (2000-07), pages 1724-1728, XP002223658 ISSN: 1079-5642

## Description

La présente invention a pour objet une nouvelle association d'un antithrombotique et d'aspirine et les compositions pharmaceutiques qui les contiennent.

Plus spécifiquement, la présente invention concerne l'association d'un antagoniste des TP-récepteurs et de l'aspirine.

Le thromboxane A₂ (TXA₂) est un métabolite instable de l'acide arachidonique qui est impliqué dans la pathogenèse de nombreux désordres de la circulation sanguine. Le thromboxane A₂ est un puissant activateur des plaquettes mais également un puissant vasoconstricteur qui possède des propriétés cellulaires prolifératives et pro-adhésives.

Le TXA₂ et d'autres métabolites de l'acide arachidonique tels que l'endoperoxyde (PGH₂), les HETE et les isoprostanes exercent leur activité par le biais de récepteurs communs nommés les TP-récepteurs.

Récemment, de nombreux travaux de recherche ont été effectués dans le but de prévenir les désordres circulatoires dus à la production excessive de thromboxane A₂. Parmi ces antagonistes, ceux décrits dans le brevet EP 648 741 et dans Bioorganic & Medicinal Chemistry Letters 8 (1998), 1381-1386 se sont avérés de puissants et sélectifs antagonistes des TP-récepteurs, actifs par voie orale et ayant une longue durée d'action.

Plus particulièrement, le composé (A) de formule (I), décrit notamment dans Bioorganic & Médicinal Chemistry Letters 8 (1998), 1375-1380 : 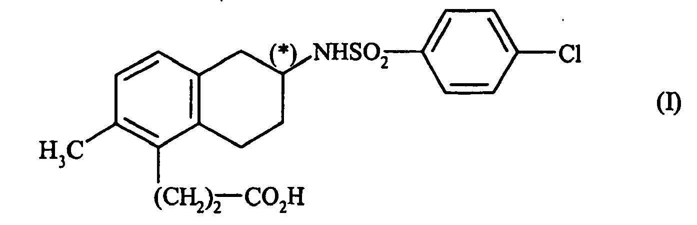 sous forme racémique ou d'isomère optiquement pur ainsi que ses sels d'addition pharmaceutiquement acceptables, s'est avéré être un puissant antithrombotique.

Ce composé inhibe sélectivement l'agrégation des plaquettes sanguines induite par une activation des TP-récepteurs. Ce composé présente de plus des propriétés antiathérosclérotiques après administration par voie orale.

Nous avons présentement découvert que l'association du composé A et de l'aspirine permettait de façon surprenante d'obtenir une synergie d'activité antithrombotique.

Il a été décrit dans la littérature que certaines associations d'agents antiagrégants plaquettaires comme le dipyridamole et l'aspirine ont des effets additifs et que cette association s'est révélée intéressante pour la prévention des accidents vasculaires cérébraux.

D'autres associations d'agents antiagrégants plaquettaires avec l'aspirine ont été décrites dans la littérature. Ces agents antiagrégants agissant sur des voies d'agrégation plaquettaire (comme les voies purinergiques, ADP) qui sont différentes de celles de l'aspirine qui agit par voie du métabolisme de l'acide arachidonique, il était attendu d'observer des effets additifs d'activité entre ces composés.

L'association, objet de la présente invention, est, quant à elle, complètement différente : le composé A et l'aspirine agissent tous les deux sur les voies du métabolisme de l'acide arachidonique : le premier agit en inhibant de manière irréversible les cyclo-oxygénases qui transforment l'acide arachidonique en endoperoxyde (PGH₂), le second agit en s'opposant à l'activité de certains métabolites de l'acide arachidonique comme le thromboxane A₂, les isoprostanes et l'endoperoxyde. L'aspirine a également été décrite comme inhibiteur de synthèse de TXA₂ (Mutschler Arzneimittelwirkungen-Lehrbuch der Pharmacologie und Toxicologie, 8. Auflage p. 231).

De manière surprenante, il s'est avéré que l'association du composé A et de l'aspirine permettait d'obtenir une importante synergie d'activité qu'aucun enseignement de la littérature ne pouvait laisser prévoir.

Cet effet synergique a été mis en évidence dans un test de thrombose artérielle chez le cobaye. Lors de ce test, il a été montré que l'activité antithrombotique du composé A est potentialisée en présence d'aspirine et augmente de manière extrêmement importante et totalement non prévisible.

Dans les associations selon l'invention, le composé (A) et l'aspirine peuvent se présenter sous forme de sels pharmaceutiquement acceptables.

Parmi les sels d'addition du composé (A), on peut citer les sels d'addition à une base pharmaceutiquement acceptable comme les sels de sodium, de potassium, de *tert*butylamine et de diéthylamine.

A titre préférentiel, le sel utilisé sera le sel de sodium.

Parmi les sels d'addition de l'aspirine, on peut citer les sels d'addition à un acide pharmaceutiquement acceptable comme les acétate, benzoate, fumarate, maléate, citrate, tartrate et le sel de lysine.

Dans les associations selon l'invention, le composé (A) possède préférentiellement la configuration absolue (R).

La présente invention concerne également les compositions pharmaceutiques renfermant une association du composé (A) et d'aspirine, éventuellement sous forme de sels pharmaceutiquement acceptables avec un ou plusieurs excipients inertes, non toxiques et appropriés.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades et gels dermiques.

La posologie est adaptable selon la nature et la sévérité de l'affection, la voie d'administration ainsi que l'âge et le poids du patient.

Dans les compositions selon l'invention, les quantités des principes actifs sont comprises entre 1 et 300 mg pour le composé (A) et entre 10 et 1000 mg pour l'aspirine.

Les compositions selon l'invention sont donc utiles pour le traitement des maladies athéro-thrombotiques dans lesquelles l'activation des TP-récepteurs et/ou la formation de métabolites sont impliqués ainsi que pour le traitement des conséquences de ces maladies. Ces pathologies incluent à titre non limitatif l'angor stable ou instable, le dysfonctionnement endothélial ou vasculaire qui accompagne des maladies comme l'hypertension, le diabète, l'insuffisance cardiaque, les troubles du système cardiovasculaire ou cérébrovasculaire, ou les troubles thrombo-emboliques associés notamment à l'athérosclérose.

Les associations selon l'invention ont été étudiées et l'effet de synergie a été mis en évidence dans un test de thrombose artérielle chez le cobaye.
Ce test est basé sur le modèle décrit initialement par Roux et Coll. (Thromb Haemost 71 : 252-256, 1994). Les cobayes sont anesthésiés par la kétamine + xylazine (90 + 12) mg/kg i.m.. La trachée est canulée et les animaux sont maintenus à respiration spontanée. La veine jugulaire est canulée, permettant l'administration intraveineuse des produits à tester. L'artère carotide est isolée, une sonde Doppler est installée permettant la mesure du flux sanguin artériel. Après stabilisation, une lésion de la paroi artérielle est crée par une pince, appliquée distale de la sonde Doppler. Suite à cette lésion, le flux sanguin diminue. Quand le flux atteint le niveau zéro, l'artère est secouée légèrement, ce qui permet de restaurer le flux. Le processus de thrombose continue conduisant de nouveau à une diminution et un arrêt du flux. Les phénomènes thrombotiques conduisent donc à un cycle de réductions de flux (CFR) qu'on observe pendant une période de 20 minutes. Après cette période, l'animal est traité ou non avec le composé (A) et on observe de nouveau pendant une période de 20 minutes les CFR. Ces expériences sont réalisées chez des animaux contrôles ou chez des animaux traités par voie intraveineuse avec de l'aspirine (2 mg/kg).

Cette étude a été réalisée avec le sel de sodium de l'isomère (R) du composé (A).

Les résultats démontrent que 10 ± 1 CFR/20 min sont observés chez les animaux non traités. Le composé (A), administré par voie intraveineuse, diminue de façon dose-dépendante les CFR ; un effet significatif est obtenu dès la dose de 0,3 mg/kg (5 ± 2 CFR/20 min). Une inhibition quasi totale (2 ± 2 CFR/20 min) est obtenue avec une dose de 1 mg/kg:
Chez les animaux traités à l'aspirine, 8 ± 1 CFR/20 min sont observés ; cette valeur n'est pas différente de celle obtenue chez les animaux contrôles.
Le composé (A), administré par voie intraveineuse chez les animaux déjà sous aspirine, diminue de façon dose dépendante les CFR ; un effet significatif est maintenant obtenu dès la dose de 0,01 mg/kg (5 ± 1 CFR/20 min), et une inhibition quasi complète est obtenue avec une dose de 0,1 mg/kg (2 ± 1 CFR/20 min).

Ces résultats démontrent d'abord la puissante activité antithrombotique du composé (A), actif dès la dose de 0,3 mg/kg.
De plus, en présence d'une dose d'aspirine telle qu'elle ne provoque pas d'effet antithrombotique, l'activité antithrombotique du composé (A) est potentialisée et augmenté d'au moins 30 fois. En effet, dès la dose de 0,01 mg/kg, cet effet est observé. Ceci signifie qu'il existe un effet de synergie très important lorsque ces deux principes actifs sont administrés simultanément.

## Revendications

1. Association du composé (A) de formule (I) éventuellement sous forme d'isomère optique ou d'un de ses sels pharmaceutiquement acceptable et d'aspirine ou d'un de ses sels pharmaceutiquement acceptable :

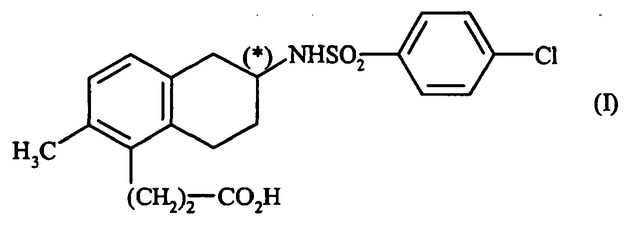

2. Association selon la revendication 1 **caractérisée en ce que** le composé (A) est sous la forme d'isomère optique de configuration (R).

3. Association selon l'une quelconque des revendications 1 ou 2 **caractérisée en ce que** le composé (A) est sous la forme d'un sel de sodium.

4. Composition pharmaceutique contenant comme principes actifs une association du composé (A) éventuellement sous forme d'isomère optique ou d'un de ses sels pharmaceutiquement acceptables et d'aspirine ou d'un de ses sels pharmaceutiquement acceptables en combinaison avec un ou plusieurs excipients ou véhicules inertes pharmaceutiquement acceptables.

5. Composition pharmaceutique selon la revendication 4 **caractérisée en ce que** le composé (A) est sous la forme d'isomère optique de configuration (R).

6. Composition pharmaceutique selon l'une quelconque des revendications 4 ou 5 **caractérisée en ce que** le composé (A) est sous forme de sel de sodium.

7. Composition pharmaceutique selon l'une quelconque des revendications 4 à 6 **caractérisée en ce que** les quantités de principes actifs sont respectivement comprises entre 1 et 300 mg pour le composé (A) et 10 et 1000 mg pour l'aspirine.

8. Composition pharmaceutique selon l'une quelconque des revendications 4 à 7 utile pour le traitement des maladies athéro-thrombotiques dans lesquelles l'activation des TP-récepteurs et/ou la formation de métabolites sont impliqués ainsi que pour le traitement des conséquences de ces maladies.

## Claims

1. Association of compound (A) of formula (I), optionally in the form of an optical isomer or one of its pharmaceutically acceptable salts, and aspirin, or one of its pharmaceutically acceptable salts :

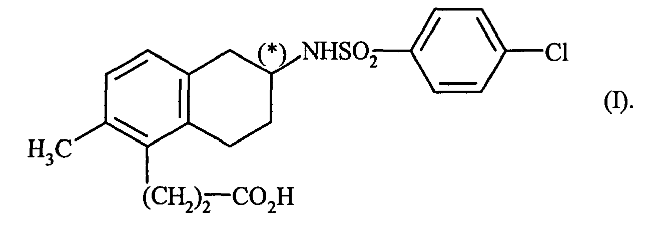

2. Association according to claim 1, **characterised in that** compound (A) is in (R) configuration optical isomer form.

3. Association according to either claim 1 or claim 2, **characterised in that** compound (A) is in the form of a sodium salt.

4. Pharmaceutical composition comprising as active ingredients an association of compound (A), optionally in the form of an optical isomer or one of its pharmaceutically acceptable salts, and aspirin, or one of its pharmaceutically acceptable salts, in combination with one or more pharmaceutically acceptable, inert excipients or carriers.

5. Pharmaceutical composition according to claim 4, **characterised in that** compound (A) is in (R) configuration optical isomer form.

6. Pharmaceutical composition according to either claim 4 or claim 5, **characterised in that** compound (A) is in sodium salt form.

7. Pharmaceutical composition according to any one of claims 4 to 6, **characterised in that** the amounts of active ingredients are in the respective ranges of from 1 to 300 mg for compound (A) and from 10 to 1000 mg for aspirin.

8. Pharmaceutical composition according to any one of claims 4 to 7, for use in the treatment of atherothrombotic illnesses involving the activation of TP receptors and/or the formation of metabolites and also in the treatment of consequences of those illnesses.

## Patentansprüche

1. Kombination der Verbindung (A) der Formel (I), gegebenenfalls in Form eines optischen Isomeren oder eines ihrer pharmazeutisch annehmbaren Salze, mit Aspirin oder einem seiner pharmazeutisch annehmbaren Salze:

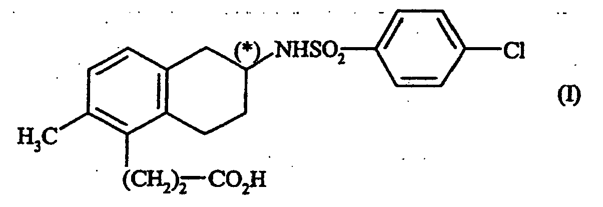

2. Kombination nach Anspruch 1, **dadurch gekennzeichnet, daß** die Verbindung (A) in Form des optischen Isomeren der Konfiguration (R) vorliegt.

3. Kombination nach einem der Ansprüche 1 oder 2, **dadurch gekennzeichnet, daß** die Verbindung (A) in Form eines Natriumsalzes vorliegt.

4. Pharmazeutische Zubereitung, enthaltend als Wirkstoffe eine Kombination der Verbindung (A), gegebenenfalls in Form eines optischen Isomeren oder eines ihrer pharmazeutisch annehmbaren Salze, mit Aspirin oder einem seiner pharmazeutisch annehmbaren Salze, zusammen mit einem oder mehreren inerten, pharmazeutisch annehmbaren Trägermaterialien oder Hilfsstoffen.

5. Pharmazeutische Zubereitung nach Anspruch 4, **dadurch gekennzeichnet, daß** die Verbindung (A) in Form des optischen Isomeren der Konfiguration (R) vorliegt.

6. Pharmazeutische Zubereitung nach einem der Ansprüche 4 oder 5, **dadurch gekennzeichnet, daß** die Verbindung (A) in Form des Natriumsalzes vorliegt.

7. Pharmazeutische Zubereitung nach einem der Ansprüche 4 bis 6, **dadurch gekennzeichnet, daß** die Mengen der Wirkstoffe für die Verbindung (A) zwischen 1 und 300 mg und für Aspirin zwischen 10 und 1000 mg liegen.

8. Pharmazeutische Zubereitung nach einem der Ansprüche 4 bis 7 nützlich zur Behandlung von athero-thrombotischen Erkrankungen, bei denen die Aktivierung der TP-Rezeptoren und/oder die Bildung von Stoffwechselprodukten beteiligt sind, sowie zur Behandlung von Folgen dieser Erkrankungen.
